# EUROPÄISCHE PATENTANMELDUNG

(11) **EP 1 138 348 A2**
(43) Veröffentlichungstag der Anmeldung: **04.10.2001**
(21) Anmeldenummer: 01107222.0
(22) Anmeldetag: 23.03.2001
(51) Int. Cl.: A61N 2/02

(54) **Magnetfeldtherapiegerät mit Fernbedieneinrichtung oder PC**

(30) Priorität: 27.03.2000 AT 5162000
(71) Anmelder: Mediscan GmbH, 4550 Kremsmünster (AT)
(72) Erfinder: Bierbaumer, Hans-Peter, Dr. h.c., 4532 Rohr/Kremstal (AT)
(74) Vertreter: Secklehner, Günter, Dr.

(57) **Zusammenfassung**

Die Erfindung betrifft eine Magnetfeldtherapievorrichtung (1), welche einen Magnetfeldgenerator (2) und eine Matte (3) umfaßt, wobei der Magnetfeldgenerator (2) für zumindest eine in oder auf einer Matte (3) angeordneten Spule (23) zur Behandlung von menschlichem und/oder tierischem und/oder pflanzlichem Gewebe ausgebildet ist und eine Steuereinrichtung (4) zur Erzeugung eines pulsierenden Stromes umfaßt und an einer Energiequelle (17) angeschlossen ist. Die Steuereinrichtung (4) weist einen digitalen Teil auf, welcher einen Mikro-kontroller (7) umfaßt, welcher seinerseits eine Speichereinrichtung (8) zu einer dauerhaften Speicherung und einem bedarfsweisen Auslesen von Impulsmustem umfaßt bzw. mit dieser in Verbindung steht und die Steuereinrichtung (4) mit einer Dateneingabe- bzw. Auswahleinrichtung (5) und einer Datenanzeige- bzw. Ausgabeeinrichtung (6) in Verbindung steht und ausgangsseitig einen Digital/Analogwandler (27) aufweist, welcher mit einer Endstufe (28) verbunden ist, welche ihrerseits ausgangsseitig mit zumindest einer Matte (3) verbindbar ist.

## Beschreibung

Die Erfindung betrifft einen Magnetfeldgenerator, eine Matte und eine Magnetfeldtherapievorrichtung, wie sie in den Ansprüchen 1, 2, 46 und 53 beschrieben sind.

Es sind bereits zahlreiche Geräte zum Erzeugen eines magnetischen Feldes für die Therapie von menschlichem, tierischem oder pflanzlichem Gewebe aus dem Stand der Technik bekannt.

Aus der DE 33 35 018 A1 ist ein Verfahren zur Steuerung eines pulsierenden Magnetfeldes, das auf ein Körperteil eines Menschen oder Tieres apliziert werden kann, bekannt. Dabei wird ein unipolares Magnetfeld erzeugt, dessen Einzelimpulse einen langsamen Anstieg und einen abrupten Abriß haben, welche von der physiologischen Pulsfrequenz getriggert werden und mit einer hohen Modulationsfrequenz, welche mit der Frequenz des unipolaren Magnetfeldes zeitlich gekoppelt ist, überlagert wird. Nachteilig bei diesem Verfahren ist, daß durch die Wahl von relativ niedrigen Frequenzen sowohl für die Grundfrequenz, als auch für die Modulationsfrequenz nicht das gesamte Spektrum der Möglichkeiten der Magnetfeldtherapie genutzt werden kann.

Aus der WO 97/46277 ist ein Elektrotherapiegerät, welches Magnetimpulse mit niedriger Frequenz verwendet, bekannt. Nachteilig ist hierbei aber, daß durch die Verwendung von niederfrequenten magnetischen Impulsen sämtliche Therapiemöglichkeiten, die nur mit hochfrequenten Magnetfeldern bzw. mit Magnetfeldern, die von einem hochfrequenten Magnetfeld überlagert werden, nicht erzielbar sind.

Aus der DE 39 25 878 A1 ist ein Magnetfeldtherapiesystem zur therapeutischen Behandlung von Nerven und Muskeln mit kurzzeitigen Magnetfeldimpulsen bekannt, welche nach einer gedämpften Schwingung abgebaut werden. Unter gedämpfter Schwingung versteht man, daß auf einen positiven Impuls ohne zeitliche Verzögerung ein negativer Impuls folgt und auf diesen ohne zeitliche Verzögerung wiederum ein positiver. Die Amplitude der unmittelbar benachbarten positiven oder negativen Impulse nimmt dabei über die Zeit betrachtet ab und erreicht schließlich den Nullpunkt. Nachteilig hierbei ist, daß bei vielen Abläufen im Stoffwechsel von Menschen, Tieren oder Pflanzen eine größere Zeitdauer beansprucht wird, wodurch sich ein ständiges Wechseln der Polarität der Magnetimpulse störend auf das Ergebnis der Behandlung auswirken kann.

Aus der WO 99/01178 ist ebenfalls ein Verfahren sowie eine Vorrichtung zur Magnetfeldtherapie bekannt. Dabei wird das magnetische Feld ebenfalls nach einer gedämpften Schwingung abgebaut, wobei Frequenzen von 34 bis 36 Hz zum Einsatz kommen. Hierbei gelten dieselben Nachteile, die schon beim Dokument DE 39 25 878 A1 angeführt wurden.

Nachteilig bei allen oben angeführten Dokumenten ist also, daß ein von einem Magnetfeldtherapiegerät abgegebenes magnetisches Feld in seiner Frequenz bzw. in der Form seiner Impulse nicht so weit variierbar ist, daß damit das gesamte Spektrum der durch Magnetfeldtherapie zu erzielenden Wirkungen abgedeckt werden kann.

Ein weiterer Nachteil der aus dem Stand der Technik bekannten Magnetfeldtherapiegeräte ist, daß die Bedienung, also die Eingabe gewünschter Parameter, wie z.B. die Behandlungsfrequenz, die Behandlungsdauer etc. und die Ausgabe von Informationen an den Benutzer bzw. an das Bedienungspersonal direkt am Gerät erfolgen muß und daher unkomfortabel ist.

Aufgabe der Erfindung ist es nunmehr, eine Magnetfeldtherapievorrichtung bzw. einen Magnetfeldgenerator zu schaffen, welches ein breites Anwendungsspektrum aufweist und für den Benutzer sehr komfortabel zu bedienen ist.

Die Aufgabe der Erfindung wird durch die Merkmale im Kennzeichenteil des Anspruches 1 gelöst.

Der sich durch die Merkmale des Kennzeichenteiles des Anspruches 1 ergebende überraschende Vorteil liegt darin, daß durch eine zumindest teilweise digitale Ausbildung der Steuereinrichtung, insbesondere durch die Verwendung eines Mikrokontrollers der Aufbau des Magnetfeldgenerators sehr einfach und kostengünstig durchführbar ist und durch die Verwendung einer Speichereinrichtung Impulsmuster dauerhaft gespeichert werden können. Ein weiterer Vorteil ist, daß durch die Ausstattung des Magnetfeldgenerators mit einer Fernbedieneinrichtung sowie einer Empfangseinrichtung hierfür eine sehr komfortable Bedienung des Magnetfeldgenerators gewährleistet ist.

Unabhängig davon wird die Aufgabe der Erfindung auch durch die Merkmale im Kennzeichenteil des Anspruches 2 gelöst.

Der sich durch die Merkmale des Kennzeichenteiles des Anspruches 2 ergebende überraschende Vorteil liegt darin, daß durch die Auslagerung der Steuereinrichtung bzw. des Mikrokontrollers des Magnetfeldgenerators in einen handelsüblichen PC die Kosten des Magnetfeldgenerators wesentlich gesenkt werden können, wodurch es möglich ist, eine Anschaffung des Magnetfeldgenerators auch für Privatpersonen sehr attraktiv zu machen. Ein weiterer Vorteil liegt darin, daß es durch die Einbindung des handelsüblichen PCs und eines Softwareprogrammes möglich ist, eine sehr gute graphische Bedienoberfläche auf einem Bildschirm des handelsüblichen PCs darzustellen, wodurch der Bedienkomfort für einen Benutzer wesentlich erhöht werden kann.

Durch eine vorteilhafte Weiterbildung gemäß Anspruch 3 wird erreicht, daß eine sehr große Speicherkapazität zur Verfügung steht, ohne weitere externe Komponenten verwenden zu müssen.

Nach einer vorteilhaften Ausgestaltung gemäß den Ansprüchen 4 und 5 werden Komponenten des handelsüblichen PCs zur Bedienung bzw. Überwachung des Magnetfeldgenerators verwendet.

Gemäß einer vorteilhaften Weiterbildung nach Anspruch 6 ist es möglich, alle weiteren Komponenten, die zum Aufbau eines Magnetfeldgenerators benötigt werden, kostengünstig auf einer Schnittstellenkarte anzuordnen, wodurch es für den Benutzer nach dem Einbau dieser Schnittstellenkarte in den PC möglich ist, den PC ohne zusätzliche externe Bauteile als Magnetfeldgenerator zu verwenden.

Eine besonders kostengünstige Ausgestaltung der Schnittstellenkarte wird nach Anspruch 7 erreicht.

Durch eine Weiterbildung gemäß Anspruch 8 wird ein Digital/Analogwandler und eine Endstufe des Magnetfeldgenerators in einer externen Komponente angeordnet und über eine serielle oder parallele Schnittstelle mit dem PC verbunden, wodurch es möglich ist, ohne hardwaremäßige Ergänzung des PCs dessen Mikrokontroller für den Magnetfeldgenerator zu nutzen.

Eine weitere vorteilhafte Ausgestaltung ist in Anspruch 9 beschrieben, wodurch es möglich ist, durch handelsübliche Datenspeicher bzw. das Internet weitere Impulsmuster in den Magnetfeldgenerator einzulesen bzw. dort zu speichern. Damit wird die Möglichkeit geschaffen, weitere Impulsmuster, welche sich an spezielle Anwendergruppen bzw. zu behandelnde Beschwerden richten, auf sehr einfache Weise zu übermitteln bzw. diesen Personenkreisen zugänglich zu machen.

Durch eine vorteilhafte Ausgestaltung gemäß Anspruch 10 und 11 wird für den Benutzer eine weitere Erhöhung des Bedienkomforts erreicht.

Durch eine vorteilhafte Weiterbildung nach Anspruch 12 wird erreicht, daß werksseitig ein oder mehrere Impulsmuster bzw. Programme in der Speichereinrichtung hinterlegt werden können, wodurch dem Benutzer die Möglichkeit gegeben wird, zwischen verschiedenen Programmen auszuwählen, womit unterschiedliche Beschwerden behandelbar sind.

Durch eine Ausgestaltung nach Anspruch 13 ist es möglich, die werksseitig fix hinterlegten Impulsmuster durch ein einfaches Softwareupdate zu ändern bzw. zu ergänzen, wodurch es sehr einfach möglich ist, den Magnetfeldgenerator mit neuen Impulsmustern bzw. Programmen auszustatten.

Durch eine Verschachtelung der werksseitig fix hinterlegten bzw. frei programmierbaren Impulsmuster nach Anspruch 14 und 15 wird in vorteilhafter Weise erreicht, daß die Wirkung des vom Magnetfeldgenerator erzeugten Magnetfeldes verstärkt wird und somit bessere Behandlungsergebnisse bzw. kürzere Behandlungszeiten erreichbar sind.

Es ist auch eine Vorgehensweise nach den Ansprüchen 16 und 17 vorteilhaft, da durch eine variable Verschachtelung der Impulsmuster besser auf die gewünschten Behandlungsergebnisse eingegangen werden kann.

Durch eine vorteilhafte Weiterbildung gemäß den Ansprüchen 18 bis 22 wird ein sehr breites Anwendungsspektrum des Magnetfeldgenerators erreicht.

Vorteilhaft ist auch eine Ausbildung gemäß den Ansprüchen 23 und 24, da dadurch eine bessere Stimulierung der Zellen des zu behandelnden Gewebes erreicht wird.

Durch eine vorteilhafte Ausgestaltung der Impulse gemäß Anspruch 25 wird eine besonders gut verträgliche Impulsform erreicht.

Weiters ist eine Ausgestaltung nach Anspruch 26 vorteilhaft, da damit ein harmonischer Verlauf der ersten Impulsgruppe erreicht wird, wodurch die Behandlungsergebnisse in positiver Weise beeinflußt werden.

Durch eine Ausgestaltung der Impulse gemäß den Ansprüchen 27 und 29 wird in vorteilhafter Weise die Überlagerung der Impulse mit einer positiven Schwingung ermöglicht, wobei den Zellen des zu behandelnden Gewebes weitere Energie zugeführt wird.

Durch eine Ausgestaltung der Impulse gemäß den Ansprüchen 28 und 29 wird in vorteilhafter Weise die Überlagerung der Impulse mit einer negativen Schwingung ermöglicht, wobei den Zellen des zu behandelnden Gewebes Energie entzogen wird, wodurch es möglich ist, auch Hitzeerkrankungen wie Fieber und dgl. mit Magnetfeldtherapie zu behandeln.

Durch eine vorteilhafte Ausgestaltung gemäß Anspruch 30 wird ein sehr großes Spektrum der Behandlungsmöglichkeiten erreicht.

Nach einer weiteren Ausführungsvariante gemäß Anspruch 31 wird das Impuls-Pausenverhältnis in vorteilhafter Weise festgelegt, wodurch die natürliche Zellfrequenz unterstützt wird und auch empfindliches Gewebe behandelbar ist.

Bei der Ausgestaltung nach den Ansprüchen 32 und 33 ist von Vorteil, daß die Ausgabe von Daten bzw. die Bestätigung von gewählten Einstellungen in für den Benutzer sehr komfortabler Form ausgebbar ist.

Durch die Verwendung von Komponenten, wie in den Ansprüchen 34 und 35 beschrieben, wird in vorteilhafter Weise erreicht, daß der Magnetfeldgenerator kostengünstig herstellbar ist.

Vorteilhaft ist weiters eine Ausgestaltung nach Anspruch 36, da durch eine Zusammenfassung der Dateneingabe bzw. Auswahleinrichtung und Datenanzeige- bzw. Ausgabeeinrichtung zu einem Gerät der Platzbedarf gesenkt werden kann und der Magnetfeldgenerator in einem optisch ansprechenden Design herstellbar ist.

Von Vorteil ist auch eine Ausbildung gemäß Anspruch 37, da dadurch eine für den Benutzer sehr einfache Möglichkeit der Dateneingabe bzw. der Auswahl gewünschter Parameter durchführbar ist und keine weiteren externen Komponenten notwendig sind.

Es erweisen sich auch Ausgestaltungen nach den Ansprüchen 38 bis 42 als vorteilhaft, da dadurch eine für den Benutzer komfortable Fernbedieneinrichtung in kostengünstiger Bauweise realisierbar ist.

Durch eine Ausgestaltung gemäß Anspruch 43 wird einem Benutzer die Möglichkeit gegeben, den Magnetfeldgenerator mit verschiedenen Matten zu betreiben.

Die Ausgestaltung nach Anspruch 44 ermöglicht in vorteilhafter Weise, daß mehrere Matten für vorzugsweise mehrere Benützer verwendet werden können, ohne dadurch die Leistungsbauteile der Endstufe stärker dimensionieren zu müssen.

Durch eine vorteilhafte Ausgestaltung gemäß Anspruch 45 wird dem Benutzer die Möglichkeit gegeben, neben der Magnetfeldtherapie auch eine Wärmebehandlung durchzuführen.

Die Aufgabe der Erfindung wird aber auch eigenständig durch die Merkmale des Anspruches 46 gelöst.

Die sich aus dem Kennzeichenteil dieses Anspruches ergebenden überraschenden Vorteile liegen darin, daß durch die Möglichkeit der Wärmebehandlung das Spektrum der zu behandelnden Beschwerden bzw. der zu erzielenden Wirkungen gegenüber der reinen Magnetfeldtherapie erhöht werden kann.

Durch die Ausgestaltung gemäß den Ansprüchen 47 und 48 wird die Spule in vorteilhafter Weise dimensioniert.

Die Ausgestaltung gemäß Anspruch 49 ermöglicht in vorteilhafter Weise den Einsatz der Matte in einem Kraftfahrzeug, wodurch dem Benutzer die Möglichkeit gegeben wird, die anregenden bzw. entspannenden Wirkungen der Magnetfeldtherapie auch während einer Autofahrt zu nützen.

Nach einer Ausgestaltung gemäß Anspruch 50 wird eine Regelung der Temperatur der Matte ermöglicht bzw. eine Überhitzung verhindert.

Durch eine vorteilhafte Ausgestaltung gemäß Anspruch 51 wird ermöglicht, die Vorteile des pulsierenden Magnetfeldes der Spule mit den Vorteilen eines permanenten Magnetfeldes eines Dauermagneten zu kombinieren.

Nach einer Ausgestaltung gemäß Anspruch 52 wird eine kompakte Einheit aus einer Matte und einem Magnetfeldgenerator geschaffen.

Die Aufgabe der Erfindung wird jedoch eigenständig auch durch die Merkmale des Anspruches 53 gelöst.

Die sich aus der Merkmalskombination des Kennzeichenteiles dieses Anspruches ergebenden überraschenden Vorteile liegen darin, daß damit eine kompakte, kostengünstige, jedoch sehr effektive Vorrichtung zur Behandlung von menschlichem und/oder tierischem und/oder pflanzlichem Gewebe durch Magnetfeldtherapie geschaffen wird.

Die Erfindung wird anschließend durch Ausführungsbeispiele näher beschrieben.

Es zeigen:
- Fig. 1: eine erfindungsgemäße Magnetfeldtherapievorrichtung, bestehend aus einem Magnetfeldgenerator und einer Matte in vereinfachter, schaubildlicher Darstellung;
- Fig. 2: einen möglichen Aufbau eines Magnetfeldgenerators in vereinfachter, schematischer Darstellung;
- Fig. 3: einen möglichen Verlauf der an die Matte angelegten Impulse und deren Verschachtelung in vereinfachter Darstellung;
- Fig. 4: einen weiteren möglichen Verlauf der an die Matte angelegten Impulse;
- Fig. 5: einen weiteren möglichen Verlauf der an die Matte angelegten Impulse;
- Fig. 6: eine weitere mögliche Ausführungsform der erfindungsgemäßen Magnetfeldtherapievorrichtung mit einem handelsüblichen PC und einer Matte in vereinfachter, schaubildlicher Darstellung.

Einführend sei festgehalten, daß in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

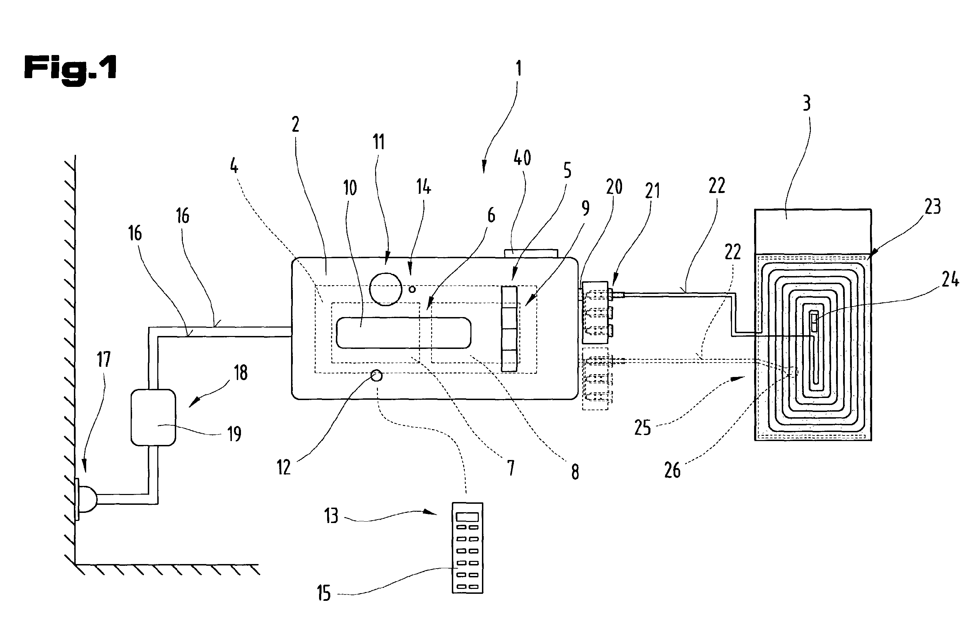

In der Fig. 1 ist eine Magnetfeldtherapievorrichtung 1, welche zumindest einen Magnetfeldgenerator 2 und eine Matte 3 umfaßt, gezeigt. Der Magnetfeldgenerator 2 umfaßt eine Steuereinrichtung 4, welche ihrerseits eine Dateneingabe- bzw. Auswahleinrichtung 5 und eine Datenanzeige- bzw. Ausgabeeinrichtung 6 umfaßt.

Die Steuervorrichtung 4 ist vorzugsweise mit einem analogen und einem digitalen Teil aufgebaut, wobei der digitale Teil einen Mikrokontroller 7 umfassen kann, welcher seinerseits mit einer Speichereinrichtung 8 in Verbindung stehen bzw. eine solche umfassen kann.

Die Dateneingabe- bzw. Auswahleinrichtung 5 kann durch einzelne Tasten oder Schalter bzw. ein Tastenfeld 9 gebildet sein. Die Datenanzeige- bzw. Ausgabeeinrichtung 6 kann durch Leuchtdioden, ein oder mehrere Siebensegmentanzeigen oder vorzugsweise einem LCD-Display 10 realisiert sein.

Selbstverständlich ist es auch möglich, die Dateneingabe- bzw. Auswahleinrichtung 5 und die Datenanzeige- bzw. Ausgabeeinrichtung 6 in einer gemeinsamen Einheit auszubilden, wie dies beispielsweise durch den Einsatz eines Touch-Screen möglich ist.

Weiters ist es möglich, daß die Datenanzeige- bzw. Ausgabeeinrichtung 6 des Magnetfeldgenerators 2 als eine Sprachausgabeeinrichtung 11 vorgesehen ist. Durch den Einsatz einer Sprachausgabeeinrichtung 11 kann in vorteilhafter Weise erreicht werden, daß Informationen über das gerade ausgeführte Programm bzw. dessen Parameter, wie etwa die Behandlungsdauer oder dgl., an einen Benutzer bzw. das Bedienungspersonal weitergegeben werden, ohne daß diese direkt auf die Datenanzeige- bzw. Ausgabeeinrichtung 6 blicken müssen.

Die Sprachausgabeeinrichtung 11 besteht vorzugsweise aus einem internen Teil, welcher im Mikrokontroller 7 bzw. in der Steuereinrichtung 4 integriert ist, und einem externen Teil, welcher vorzugsweise durch einen Verstärker und einen Lautsprecher zum Verstärken bzw. Ausgeben der aus der Speichereinrichtung 8 ausgelesenen und in den Mikrokontroller 7 bzw. die Steuereinrichtung 4 eingelesenen Sprachinformationen gebildet ist.

Selbstverständlich ist es möglich, diese Sprachausgabeeinrichtung 11 für die Ausgabe verschiedener Sprachen auszubilden.

Die Steuereinrichtung 4 kann weiters mit einer Empfangseinrichtung 12 für eine Fernbedieneinrichtung 13 in Verbindung stehen.

Dabei kann die Fernbedieneinrichtung 13 auch durch eine Spracheingabeeinrichtung 14 realisiert sein. In diesem Fall umfaßt die Empfangseinrichtung 12 ein Mikrofon oder dgl. zur Aufnahme der von einem Benutzer bzw. vom Bedienungspersonal ausgesprochenen akustischen Befehle und einem Verstärker bzw. eine Umwandeleinrichtung zum Verstärken bzw. Anpassen dieser Informationen an für die Steuereinrichtung 4 bzw. den Mikrokontroller 7 verarbeitbare Signale.

Damit kann erreicht werden, daß Änderungen von verschiedenen Parametern, wie z.B. Behandlungsdauer und/oder Intensität und/oder der Ablauf verschiedener Programmschritte usw. von einem Benutzer bequem durchgeführt werden können, ohne daß dieser direkte Eingaben am Gerät vornehmen muß.

Diese Spracheingabeeinrichtung 14 ist in vorteilhafter Weise in unterschiedlichen Staaten durch unterschiedliche Sprachen realisiert.

Zusätzlich oder optional zur Spracheingabeeinrichtung 14 kann die Fernbedieneinrichtung 13 mit einem externen Bedienelement 15 aufgebaut sein. Die Signalübertragung zwischen dem externen Bedienelement 15 und der Empfangseinrichtung 12 kann dabei vorzugsweise über ein Infrarotsignal erfolgen. Zu diesem Zweck ist das externe Bedienelement 15 mit einem Infrarotsender und die Empfangseinrichtung 12 mit einem Infrarotempfänger ausgestattet.

Es ist aber ebenfalls möglich, die Signalübertragung zwischen der Fernbedieneinrichtung 13 und der Empfangseinrichtung 12 über Funk, Ultraschall oder dgl. durchzuführen.

Weiters ist es möglich, die Signalübertragung zwischen der Fernbedieneinrichtung 13 und der Empfangseinrichtung 12 auch mittels eines Kabels bzw. eines Bussystemes durchzuführen.

Das externe Bedienelement 15 kann einzelne Taster bzw. ein Tastenfeld umfassen. Diese können durch alle aus dem Stand der Technik bekannten Tast- bzw. Schalteinrichtungen, wie z.B. Folientaster, gebildet sein. Weiters ist es möglich, daß das externe Bedienelement 15 ein Display aufweist, welches aus Siebensegmentanzeigen bzw. einem LCD-Display oder dgl. gebildet ist.

Zur Energieversorgung ist der Magnetfeldgenerator 2 über Anschlußleitungen 16 mit einer Energiequelle 17 verbunden. Diese Energiequelle 17 kann durch eine Gleich- oder Wechselspannungsquelle, wie beispielsweise ein 110 V bzw. ein 230 V Wechselspannungsnetz, wie es für den Gebrauch in Haushalten vorgesehen ist, oder durch ein 12 V bzw. 24 V aufweisendes Gleichspannungsnetzteil, wie es beispielsweise in Kraftfahrzeugen eingesetzt wird, gebildet sein.

Zum kuppelbaren Anschluß des Magnetfeldgenerators 2 an die Energiequelle 17 kann am Ende der Anschlußleitungen 16 eine für den jeweiligen Verwendungszweck geeignete Kupplungseinrichtung vorhanden sein. Für den Einsatz in einem Kraftfahrzeug kann ein 12 V Adapter vorhanden sein, für den Einsatz im Haushalts- bzw. Therapiebereich kann ein 110 V bzw. 230 V Netzstecker ausgeführt sein.

Zur Umformung der von der Energiequelle 17 bereitgestellten Energie ist es möglich, eine Umwandlungseinrichtung 18, vorzugsweise einen Transformator 19 einzusetzen. Die Umwandlungseinrichtung 18 bzw. der Transformator 19 kann dabei im Magnetfeldgenerator 2 integriert sein, es ist aber ebenfalls möglich, diesen extern auszuführen oder handelsübliche Kleinnetzteile bzw. Steckernetzteile zu verwenden.

Beim Einsatz der Magnetfeldtherapievorrichtung 1 mit Gleichspannungsquellen, z.B. in Kraftfahrzeugen, kann diese Umwandlungseinrichtung 18 auch wegfallen.

Am Magnetfeldgenerator 2 kann eine Ausgangsbuchse 20 vorhanden sein, die über einen korrespondierenden Stecker 21 den kuppelbaren Anschluß einer zu einer Matte 3 führenden Leitung 22 ermöglicht.

Selbstverständlich ist es aber auch möglich, am Magnetfeldgenerator 2 mehrere Ausgangsbuchsen 20 vorzusehen, um damit die Möglichkeit zu schaffen, mehrere Matten 3 mit dem gleichen Magnetfeldgenerator 2 zu versorgen. Damit wird es nun möglich, gleichzeitig mehrere Patienten mit einem Magnetfeldgenerator 2 zu behandeln, oder es können verschiedene Regionen eines Patienten durch die Verwendung von mehreren Matten 3 bzw. Kissen behandelt werden.

Um eine Leistungsausbeute des Magnetfeldgenerators 2 zu erhöhen, ist es möglich, die Energiebeaufschlagung der einzelnen Matten 3 zeitlich voneinander versetzt durchzuführen, d.h. also, während die eine Matte 3 mit Energie beaufschlagt wird, befindet sich die andere Matte 3 gerade in einem Zeitabschnitt, in dem keine Energie zugeführt wird. Auf diese Weise ist es beispielsweise möglich, Leistungsbauteile des Magnetfeldgenerators 2 für zwei Matten 3 auszulegen und drei Matten 3 zu betreiben.

Es ist aber ebenso möglich, zwischen der bzw. den Ausgangsbuchsen 20 und den Steckern 21 einen zusätzlichen Adapter anzuordnen, welcher die Möglichkeit bietet, mehrere Matten 3 mit mehreren Steckern 21 an einer Ausgangsbuchse 20 anzuschließen. Die Verdrahtung in diesem zusätzlichen Adapter kann so ausgebildet sein, daß sich daraus sowohl eine serielle Schaltung der Matten 3 als auch eine Parallelschaltung der Matten 3 ergibt. Durch die Verwendung des zusätzlichen Adapters kann die Möglichkeit geschaffen werden, mehrere Matten 3 mit einem Magnetfeldgenerator 2 zu betreiben, welche gleichzeitig mit Energie beaufschlagbar sind.

Als Einsatzgebiet sei dabei nur beispielsweise die Möglichkeit erwähnt, mehrere Sportler mit Hilfe von Magnetfeldtherapie gleichzeitig aufzuwärmen.

Die Matte 3 ist vorzugsweise flexibel ausgebildet und weist eine für das zu behandelnde Gewebe geeignete Größe auf. Vorzugsweise ist die Matte zum Behandeln von menschlichem Gewebe ausgebildet und weist eine Größe von etwa 0,8 x 1,8 m auf, es ist aber ebenfalls möglich, die Matte 3 zum Behandeln von Tieren, wie beispielsweise Haustieren oder Pferden und dgl. oder für den Einsatz an pflanzlichem Gewebe vorzusehen und in einer dazu geeigneten Größe auszubilden.

Für den Aufbau eines magnetischen Feldes weist die Matte 3 zumindest eine Spule 23 auf, die im Bereich des zu behandelnden Gewebes angeordnet ist. Die Windungszahl der Spule 23 liegt vorzugsweise bei 10 bis 40 Windungen, welche in oder auf der Matte 3 angeordnet sein können.

Für die Behandlung von Menschen kann sich die Spule 23 über etwa 4/5 der Gesamtlänge der Matte 3 erstrecken. Damit wird in vorteilhafter Weise erreicht, daß der gesamte menschliche Organismus mit Ausnahme des Kopfbereiches in die Magnetfeldtherapie einbezogen werden kann.

Für die gezielte Behandlung von einzelnen Körperteilen bzw. Organen ist es jedoch auch möglich, die Matte 3 mit anderen Abmessungen, z.B. als Kissen, auszuführen.

Da auch an den Einsatz der Magnetfeldtherapievorrichtung 1 in einem Kraftfahrzeug gedacht wird, ist es möglich, die Matte 3 so auszustatten, daß diese leicht über einen Sitz eines Kraftfahrzeuges gelegt werden kann oder diese auch als Sitzüberzug ausgebildet ist.

Weiters ist es möglich, in der Matte 3 mehrere Spulen 23 auszuführen und somit eine gezielte Behandlung von unterschiedlichen Regionen durchzuführen. Zur Unterstützung des magnetischen Feldes ist es auch möglich, neben der bzw. den Spulen 23 einen bzw. mehrere Dauermagnete 24, vorzugsweise in den Zentren der Spulen 23, anzuordnen.

Da es sich bei der Behandlung zahlreicher körperlicher Beschwerden von Patienten, wie z.B. Gicht, Rheuma und Stoffwechselerkrankungen sehr vorteilhaft erwiesen hat, auch eine Wärmebehandlung durchzuführen, kann in der Matte 3 ein Heizungskreis 25 vorgesehen sein. Dieser Heizungskreis 25 wird dabei vorzugsweise durch eine Widerstandsfolie, insbesondere einer Kohleschichtfolie, gebildet, da damit die Flexibilität und das Gewicht der Matte 3 nicht negativ beeinflußt wird.

Weiters ist es möglich, die Matte 3 mit mehreren Heizungskreisen 25 auszustatten, welche mit unterschiedlichen Temperaturen betrieben werden können. Dadurch kann erreicht werden, daß verschiedene Körperregionen eine Wärmebehandlung erfahren, jedoch nicht die gesamte Matte beheizt werden muß.

Die Beaufschlagung dieses oder dieser Heizungskreise 25 mit Energie kann entweder durch den Benutzer am Magnetfeldgenerator 2 bzw. mit der Spracheingabeeinrichtung 14 oder dem externen Bedienelement 15 durchgeführt werden. Es ist jedoch ebenfalls möglich, daß bei verschiedenen im Magnetfeldgenerator 2 hinterlegten Programmen der oder die Heizungskreise 25 automatisch mit Energie versorgt werden.

Zur Regelung der Temperatur der Matte 3 ist es möglich, den Heizungskreis 25 mit einem Thermostat oder einem Temperatursensor 26 auszustatten. Die Temperaturwahl kann dabei entweder manuell an der Dateneingabe- bzw. Auswahleinrichtung 5 des Magnetfeldgenerators 2 eingestellt werden oder es erfolgt eine automatische Temperaturwahl und Regelung durch die Steuereinrichtung 4 des Magnetfeldgenerators 2.

Selbstverständlich können aber auch alle aus dem Stand der Technik bekannten Wärmeerzeugungskomponenten, wie z.B. Heizwendeln und dgl., für den Aufbau des Heizungskreises 25 in der Matte 3 herangezogen werden.

Die Versorgung des bzw. der Heizungskreise 25 erfolgt vorteilhafterweise in derselben mehrpoligen Leitung 22, mit der auch die Spule 23 mit Energie versorgt wird. Es ist aber auch möglich, eine weitere Leitung 22 für die Versorgung des Heizungskreises 25 vom Magnetfeldgenerator 2 zur Matte 3 vorzusehen, wie dies strichliert dargestellt ist.

Die Heizleistung des Heizungskreises 25 kann bis zu 50 Watt betragen, welche vorzugsweise von der Steuereinrichtung 4 des Magnetfeldgenerators 2 in Form einer Gleichspannung mit bis zu 50 V, vorzugsweise bis zu 24 V zugeführt wird.

Selbstverständlich ist es auch möglich, zwei getrennte Matten 3 übereinander anzuordnen, wobei dann eine Matte 3 die Aufgabe der Wärmebehandlung übernimmt und die andere Matte 3 für die Magnetfeldtherapie ausgebildet ist. Der Vorteil einer Ausgestaltung mit zwei Matten 3 ist, daß dadurch handelsübliche Matten für die Magnetfeldtherapie bzw. Heizmatten, wie sie aus dem Stand der Technik in vielfältiger Form bekannt sind, zum Einsatz kommen können.

Die Versorgung der Matte 3, welche den Heizungskreis 25 aufweist, kann in derselben mehrpoligen Leitung 20 erfolgen, mit der auch die Matte 3, welche die Spule 23 aufweist, versorgt wird. Es ist aber ebenfalls möglich, für die beiden getrennten Matten 3 getrennte Leitungen 22 zu deren Energieversorgung vom Magnetfeldgenerator 2 vorzusehen.

Indes ist es auch möglich, den Magnetfeldgenerator 2 nur an die Matte 3 anzuschließen, welche die Spule 23 aufweist und somit auf die Wärmebehandlung zu verzichten.

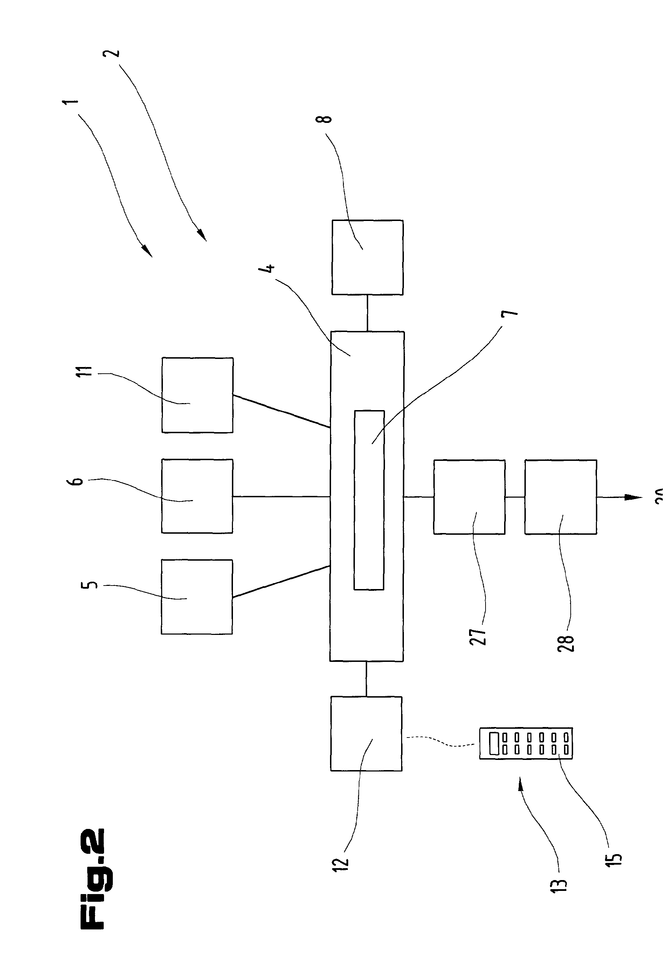

In Fig. 2 ist ein mögliches Blockschaltbild des Magnetfeldgenerators 2 gezeigt. Dabei weist der Magnetfeldgenerator 2 neben der Dateneingabe bzw. Auswahleinrichtung 5 der Datenanzeige- bzw. Ausgabeeinrichtung 6 und der Sprachausgabeeinrichtung 11 eine Fernbedieneinrichtung 13 mit einem externen Bedienelement 15 und eine Empfangseinrichtung 12 auf. Sämtliche von diesen Einrichtungen generierten bzw. an diese ausgegebene Signale werden von der Steuereinrichtung 4 bzw. vom Mikrokontroller 7 verarbeitet bzw. von dieser (diesem) ausgegeben. Die Steuereinrichtung 4 umfaßt vorzugsweise einen Mikrokontroller 7, welcher mit einer Speichereinrichtung 8 verbunden ist bzw. eine Speichereinrichtung 8 umfaßt.

Zur Signalaufbereitung der dem Mikrokontroller 7 zugeführten Signale ist es möglich, daß die Steuereinrichtung 4 Umwandelorgane zur Umwandlung der von bzw. an die Dateneingabe-bzw. Auswahleinrichtung 5 und/oder Datenanzeige- bzw. Ausgabeeinrichtung 6 und/oder Sprachausgabeeinrichtung 11 und/oder Empfangseinrichtung 12 und/oder Fernbedieneinrichtung 13 empfangenen bzw. geschickten Daten umfaßt, welche beispielsweise durch Analog/Digitalwandler, Digital/Analogwandler, Verstärker, Bildschirmtreiber und dgl. gebildet sein können, der Übersichtlichkeit halber aber nicht dargestellt wurden.

Weiters wurde aus Gründen der Übersichtlichkeit in Fig. 2 die Energieversorgung des Magnetfeldgenerators 2 von der Energiequelle 17 über Anschlußleitungen 16 nicht dargestellt.

An einem Ausgang des Mikrokontrollers 7 bzw. der Steuereinrichtung 4 ist ein Digital/ Analogwandler 27 angeordnet, welcher die Ausgangssignale des Mikrokontrollers 7 in für eine Endstufe 28 verarbeitbare analoge Signale umwandelt. Die Endstufe 28 ist zur Generierung eines für die Magnetfeldtherapie benötigten Stromes bzw. einer Spannung aufgebaut und verstärkt die vom Digital/Analogwandler 27 erhaltenen Signale, insbesondere Impulsmuster, um einen vorbestimmten oder variablen Wert. Da für die Entstehung eines Magnetfeldes der Strom von ausschlaggebender Bedeutung ist, wird vorzugsweise mit einem "eingeprägten Strom" gearbeitet.

In der Speichereinrichtung 8 sind zur Behandlung verschiedener physischer bzw. psychischer Erkrankungen oder zur Steigerung des Stoffwechsels oder allgemeinen Wohlbefindens und dgl. verschiedene Programme in digitaler Form hinterlegt. Diese Programme können sich aus Impulsmustern, wie sie nachstehend noch näher beschrieben werden, Sprachinformationen für die Sprachausgabeeinrichtung 11 und gegebenenfalls Daten zur Energiebeaufschlagung des bzw. der Heizungskreise 25 zusammensetzen.

Es besteht nun für einen Benutzer die Möglichkeit, über die Dateneingabe- bzw. Auswahleinrichtung 5 und/oder die Fernbedieneinrichtung 13 ein gewünschtes Programm aufzurufen, welches dann aus der Speichereinrichtung 8 ausgelesen und in die Steuereinrichtung 4 bzw. den Mikrokontroller 7 geladen wird, welche anschließend ein dementsprechendes Signal an den Digital/Analogwandler 27 weitergibt, welcher das digitale Signal vom Mikrokontroller 7 in ein für die Endstufe 28 verarbeitbares analoges Signal umwandelt und dieser zuführt. Die Endstufe 28 verstärkt dieses Signal und leitet es über die Leitung 22 an die Matte 3 bzw. die Spule 23 der Matte 3 weiter. Bei dem von der Endstufe 28 ausgegebenen Signal handelt es sich vorzugsweise um einen eingeprägten Strom, der eine Amplitude bis etwa 1,5 A aufweist.

Bei der Auswahl eines Programmes wird zur Bestätigung von der Steuervorrichtung 4 bzw. vom Mikrokontroller 7 ein entsprechendes Signal an der Datenanzeige- bzw. Ausgabeeinrichtung 6 bzw. der Sprachausgabeeinrichtung 11 ausgegeben. Die Datenanzeige- bzw. Ausgabeeinrichtung 6 kann weiters ein oder mehrere noch zu ändernde Parameter, auf die weiter hinten näher eingegangen wird, anzeigen. Diese Parameter können an der Dateneingabe- bzw. Auswahleinrichtung 5 noch innerhalb von vorgegebenen Grenzen verändert werden. Durch Betätigung einer Starttaste an der Dateneingabe- bzw. Auswahleinrichtung 5 bzw. eine Freigabe über die Fernbedieneinrichtung 13 ist es nun möglich, das ausgewählte Programm zu starten.

Zusätzlich zu den werksseitig vorgesehenen Programmen kann die Möglichkeit bestehen, über einen "Teaching Modus" verschiedene persönliche Einstellungen über die Dateneingabe-bzw. Auswahleinrichtung 5 in die Steuereinrichtung 4 einzugeben bzw. in der Speichereinrichtung 8 dauerhaft zu hinterlegen und zu einem späteren Zeitpunkt ohne größere Einstellarbeiten wieder aufzurufen.

In der Speichereinrichtung 8 sind weiters Sprachmuster für die Sprachausgabeeinrichtung 11 gespeichert. Für diesen Zweck ist es selbstverständlich auch möglich, eine zweite Speichereinrichtung 8 vorzusehen. Weiters ist es möglich, die Daten der Sprachausgabeeinrichtung 11 in verschiedenen Sprachen zu hinterlegen, um das Gerät damit bedienungsfreundlicher zu machen.

Nach dem Einschalten des Magnetfeldgenerators erfolgt also vorzugsweise eine Abfrage, in welcher Sprache die Datenein- bzw. -ausgabe an der Dateneingabe- bzw. Auswahleinrichtung 5 und der Datenanzeige- bzw. Ausgabeeinrichtung 6 bzw. der Spracheingabe 14 und der Sprachausgabeeinrichtung 11 erfolgen soll. Daraufhin werden dementsprechende Daten aus der Speichereinrichtung 8 ausgelesen und der Steuereinrichtung 4 übermittelt, damit diese eine Konversation in der gewünschten Sprache durchführen kann.

Für den Erfolg einer Behandlung mit Magnetfeldern hat es sich als vorteilhaft herausgestellt, den an die Spule 23 (siehe Fig. 1) angelegten Strom pulsierend auszugestalten und diese Impulse in einem bestimmten nachfolgend beschriebenen Muster zu "verschachteln" bzw. zu Gruppen zusammenzufassen und diese Gruppen hintereinander zu schalten.

Ebenfalls hat es sich als vorteilhaft erwiesen, die Polarität der Stromimpulse über einen längeren Zeitraum, vorzugsweise zwischen 10 und 30000 Sekunden, beizubehalten und anschließend dieselben Impulse mit umgekehrter Polarität an die Spule 23 der Matte 3 anzulegen.

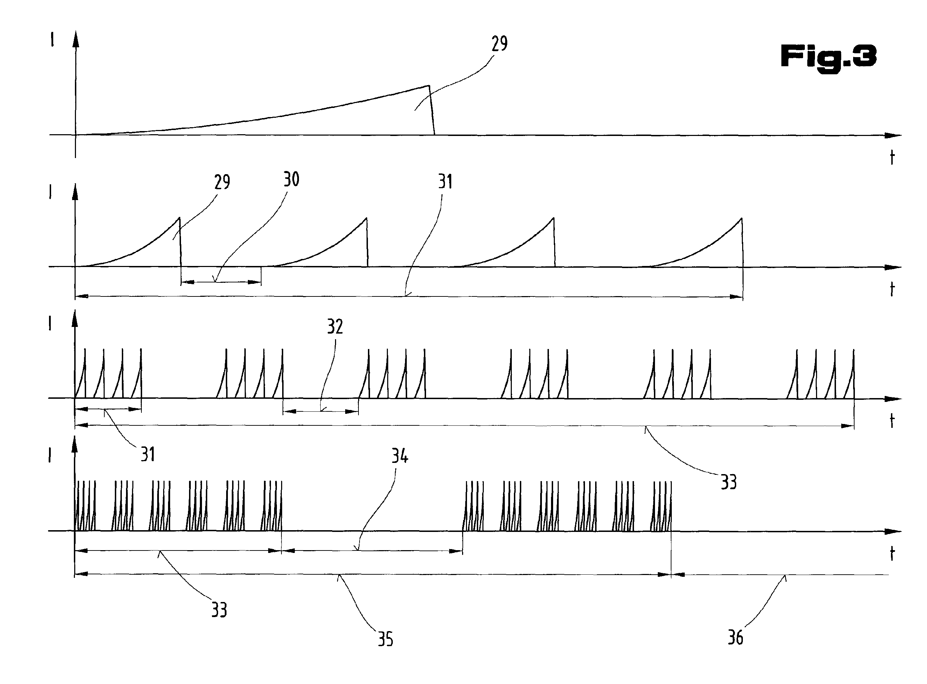

In der Fig. 3 ist ein möglicher Verlauf eines vom Magnetfeldgenerator 2 erzeugten und an die Matte 3 (siehe Fig. 1) angelegten Stromes dargestellt. Dabei weisen Impulse 29 eine vierfache "Verschachtelung" auf. Diese Impulse 29 können sägezahnförmig aufgebaut sein, d.h. die steigende Flanke kann flach ausgebildet sein, während die fallende Flanke einen sehr steilen Verlauf haben kann. Es ist ebenfalls möglich, die steigende Flanke nicht linear auszubilden, sondern diese mit beliebiger Krümmung ansteigen zu lassen. Auf diesen Impuls 29 folgt eine Pause 30.

Nun werden eine variierbare Anzahl an Impulsen 29 und Pausen 30 hintereinander gesetzt und bilden so eine erste Impulsgruppe 31. Nach dieser ersten Impulsgruppe 31 erfolgt wiederum eine Pause 32. Die Anzahl der Impulsen 29 und Pausen 30, die zu einer ersten Impulsgruppe 31 zusammengefaßt werden, kann zwischen eins und hundert liegen.

Eine variierbare Anzahl von ersten Impulsgruppen 31 und Pausen 32 werden nun zu einer zweiten Impulsgruppe 33 zusammengefaßt. Nach dieser Impulsgruppe 33 wird eine Pause 34 eingehalten. Die Anzahl der ersten Impulsgruppen 31 und Pausen 32, die zu einer zweiten Impulsgruppe 33 zusammengefaßt werden, kann ebenfalls zwischen eins und hundert betragen.

Nun werden eine variierbare Anzahl von zweiten Impulsgruppen 33 und darauffolgende Pausen 34 zu einer dritten Impulsgruppe 35 zusammengefaßt. Nach dieser dritten Impulsgruppe 35 erfolgt eine Pause 36. Die Anzahl der zweiten Impulsgruppen 33 und darauffolgenden Pausen 34, die zu einer großen Impulsgruppe 35 zusammengefaßt werden, beträgt ebenfalls zwischen eins und hundert.

Nun wird eine variierbare Anzahl, vorzugsweise zwischen eins und hundert, von diesen dritten Impulsgruppen 35 und Pausen 36 an die Matte 3 angelegt. Nach der vollständigen Ausführung der dritten Impulsgruppe 35 und der Pause 36 kann vorzugsweise die Polarität des an der Matte 3 angelegten Stromes umgekehrt werden und eine weitere dritte Impulsgruppe 35 sowie eine Pause 36 mit entgegengesetzter Polarität an die Matte 3 angelegt werden.

Bei einer höheren Verschachtelung der Impulse 29 bzw. der ersten, zweiten und dritten Impulsgruppen 31, 33, 35 kann die Anzahl der höheren Impulsgruppe, welche mit darauffolgenden Pausen zu einer noch höheren Impulsgruppe zusammengefaßt werden, jeweils zwischen eins und hundert gewählt werden.

In der Fig. 3 werden vier Impulse 29 mit den dazugehörigen Pausen 30 zu einer ersten Impulsgruppe 31 zusammengefaßt. Weiters werden sechs von diesen ersten Impulsgruppen 31 mit ihren dazugehörigen Pausen 32 zu einer zweiten Impulsgruppe 33 zusammengefaßt. Zur Entstehung einer dritten Impulsgruppe 35 werden zwei zweite Impulsgruppen 33 und eine dazugehörige Pause 34 zusammengefaßt. Nachfolgend an die große Impulsgruppe 35 erfolgt eine Pause 36. Bei den in Fig. 3 dargestellten Diagrammen wurden zur besseren Übersicht die Maßstäbe der Zeitachsen immer weiter verkleinert, um einen gesamten möglichen Verlauf eines an die Spule 23 der Matte 3 angelegten Stromes darstellen zu können.

Die Impulse 29 bzw. Impulsgruppen 31, 33, 35 können zu ihren dazugehörigen Pausen 30, 32, 34, 36 in einem bestimmten Verhältnis stehen. Dieses Verhältnis kann 1,618 betragen, d.h. also, daß die Länge eines Impulses 29 1,618 mal länger ist als die Pause 30. Gleichzeitig kann gesagt werden, daß die Pause 30 0,618 mal kürzer ist als die Länge des Impulses 29. Dasselbe kann auch zwischen der ersten Impulsgruppe 31 und der Pause 32 bzw. der zweiten Impulsgruppe 33 und der Pause 34 bzw. der dritten Impulsgruppe 35 und der Pause 36 bestehen. Dieses spezielle Verhältnis wird als "goldener Schnitt" bezeichnet und unterstützt den natürlichen Stoffwechsel der Zellen, wodurch eine raschere Heilung von stoffwechselbedingten Erkrankungen erzielt wird.

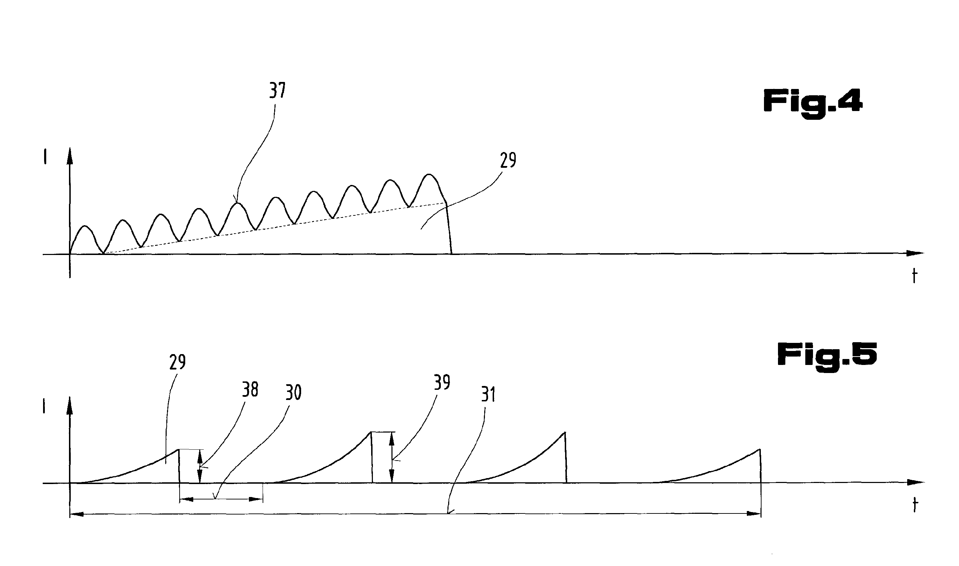

In Fig. 4 ist eine mögliche Form des an die Matte 3 angelegten Impulses 29 gezeigt. Der Impuls 29 weist zusätzlich zu seinem sägezahnförmigen Verlauf überlagerte Schwingungen 37 auf. Diese Schwingungen 37 können wie gezeigt durch positive Halbwellen eines gleichgerichteten sinusförmigen Stromes ausgeführt sein. Dieser Betriebszustand wird als "Yang" bezeichnet. Er regt die Yang-Meridiane an und führt der zu behandelnden Zelle zusätzliche Energie zu.

Es ist jedoch ebenfalls möglich, diese Schwingungen 37 durch negative Halbwellen eines gleichgerichteten sinusförmigen Stromes auszuführen. Dieser Betriebszustand wird als "Yin" bezeichnet und regt die Yin-Meridiane an. Damit ist es möglich, die Zellenergie zu senken und es wird die Möglichkeit geschaffen, auch Hitzeerkrankungen wie Fieber und dgl. mit Magnetfeldtherapie zu behandeln.

Die Anzahl dieser positiven oder negativen Schwingungen 37 auf einem Impuls 29 kann zwischen 1 und 100 gewählt werden (in Fig. 4 ist ein Verlauf des Impulses 29 mit 10 Schwingungen 37 gezeigt).

Eine Amplitude dieser positiven oder negativen Halbwellen der Schwingungen 37 auf dem Impuls 29 kann vorzugsweise konstant gehalten werden, es ist jedoch ebenfalls möglich, die Amplitude unmittelbar aufeinander folgender Halbwellen zu senken oder ansteigen zu lassen. Sie kann etwa 0 bis 0,5 Ampere betragen.

Weiters ist es aber auch möglich, die Schwingung 37 nicht mit positiven oder negativen Halbwellen eines gleichgewichtigen Stromes auszugestalten, sondern einen sinusförmigen, dreieckförmigen, rechteckförmigen oder dgl. Verlauf zu wählen.

Die weitere Verschachtelung der Impulse 29 kann, wie schon in Fig. 3 beschrieben, erfolgen.

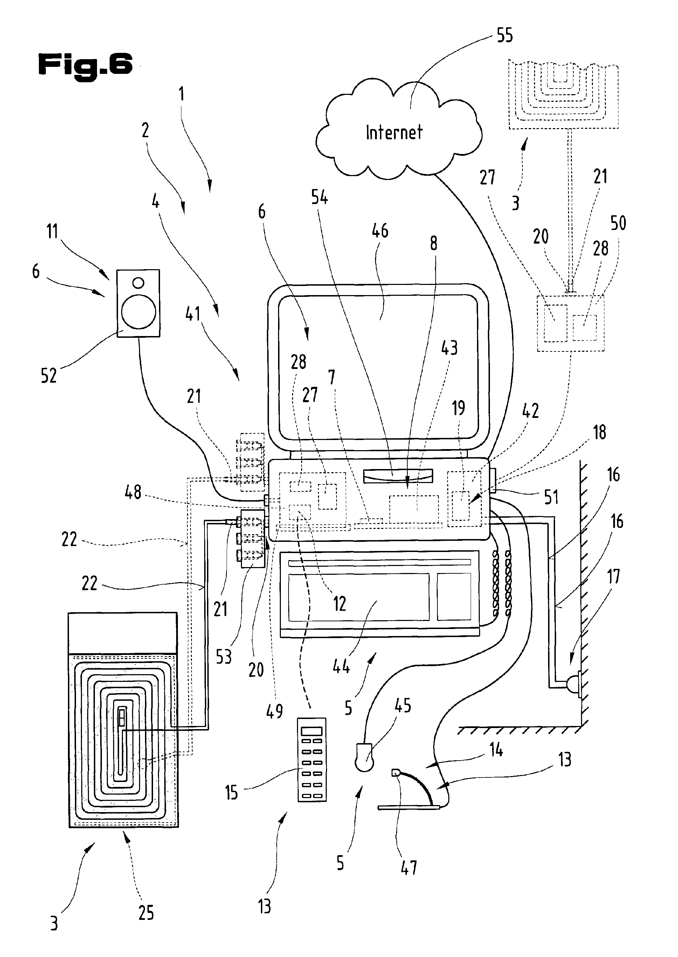

In Fig. 5 ist ebenfalls ein möglicher Verlauf des Stromes, der an die Matte 3 (siehe Fig. 1) angelegt wird, gezeigt. Es kann dieselbe "Verschachtelung" gelten, wie schon in Fig. 3 beschrieben. Ebenfalls können auch Schwingungen 37, wie in Fig. 4 beschrieben, verwendet werden. In diesem Ausführungsbeispiel sind jedoch die Amplituden der unmittelbar aufeinanderfolgenden Impulse 29 nicht immer gleich groß. Zumindest der jeweils erste und letzte Impuls 29 der ersten Impulsgruppe 31 weist eine geringere Amplitudenhöhe 38 auf als die Amplitudenhöhe 39 aller übrigen Impulse 29 der ersten Impulsgruppe 31.

Es ist aber auch möglich, die Amplitudenhöhe 38 der unmittelbar aufeinanderfolgenden Impulse 29 der ersten Impulsgruppe 31 ansteigend auszubilden, bis eine Amplitudenhöhe 39 erreicht ist und die letzten unmittelbar aufeinanderfolgenden Impulse 29 wieder mit der geringeren Amplitudenhöhe 38 auszugestalten.

Die Frequenz der Impulse 29 in den Fig. 3 bis 5 kann von 0,02 Hz bis etwa 80 kHz betragen.

Die Form der Impulse 29 muß nicht, wie dargestellt, sägezahnförmig verlaufen, es ist ebenfalls möglich, diese dreieckförmig auszubilden.

Durch die Änderung der Anzahl der Impulse 29 bzw. der Anzahl der ersten Impulsgruppen 31 bzw. zweiten Impulsgruppen 33 bzw. dritten Impulsgruppen 35 bzw. der Schwingungen 37 ist es nun möglich, unterschiedliche Beschwerden zu lindern bzw. das zu behandelnde Gewebe anzuregen. Mit den in den Fig. 3 bis 5 dargestellten Wellenformen konnten folgende therapeutische Möglichkeiten nachgewiesen werden: Es kommt zu einem Krippeln im behandelten Gewebe, die Durchblutung kann gefördert werden, es werden sowohl der abbauende als auch der aufbauende Stoffwechsel beschleunigt, es kommt zu einer Beschleunigung der Verdauung, es kommt zu einer beschleunigten Abheilung von Wunden bzw. Verletzungen und erstmalig konnten Hitzeerkrankungen wie Fieber behandelt werden. Viele Testpersonen sprachen von einem deutlich spürbaren Entspannungsgefühl bzw. von einem Durchfließen des zu behandelnden Körperteiles mit Energie.

Die in den Fig. 3 bis 5 gewählte vierfache Verschachtelung wurde nur beispielsweise herangezogen. Es ist ebenfalls möglich, die Impulse 29 weniger oft oder öfters zu "verschachteln". Eine vorteilhafte "Verschachtelung" liegt zwischen ein und acht Mal.

In der Speichereinrichtung 28 des Magnetfeldgenerators 2 können nun mehrere unterschiedliche Programme, vorzugsweise zwischen 1 und 15, zur unterschiedlichen Behandlung hinterlegt sein. Diese Programme unterscheiden sich durch folgende Merkmale:
- die Frequenz der Impulse 29
- die Länge der Pause 30
- die Anzahl der Impulse 29, die zu einer ersten Impulsgruppe 31 zusammengefaßt werden
- die Länge der Pause 32
- die Anzahl der ersten Impulsgruppen 31, die zu einer zweiten Impulsgruppe 33 zusammengefaßt werden
- die Länge der Pausen 34
- die Anzahl der zweiten Impulsgruppen 33, die zu dritten Impulsgruppen 35 zusammengefaßt werden
- die Länge der Pausen 36
- die Anzahl der Schwingungen 37 der Impulse 29
- ob positive oder negative Halbwellen für die Schwingung 37 verwendet werden. (Yin- oder Yang-Betriebszustand)
- ob alle Impulse 29 die selbe Amplitude aufweisen oder ob zumindest die ersten und letzten Impulse 29 der ersten Impulsgruppe 31 jeweils eine geringere Amplitude 38 aufweist
- ob zusätzlich zur Magnetfeldenergie auch Wärmeenergie zugeführt wird
- den Temperaturwert, auf den die Matte 3 geregelt wird
- beim Vorhandensein von mehreren Heizungskreisen 25, ob diese auf unterschiedliche Temperaturwerte geregelt werden.

Weiters ist es möglich, daß der Magnetfeldgenerator 2 eine serielle, parallele oder eine andere aus dem Stand der Technik bekannte Schnittstelle 40 (siehe Fig. 1) aufweist, welche zur Übertragung von Daten aus einer übergeordneten Computeranlage, vorzugsweise einem PC oder einem Laptop, zur Steuereinrichtung 4 bzw. dem Mikrokontroller 7 dient. Durch diese Schnittstelle 40 wird es möglich, Änderungen und/oder Ergänzungen der werksseitig fix in der Speichereinrichtung 8 hinterlegten Impulsmuster durchzuführen. Dies kann in einfacher Weise durch ein Softwareupdate durchgeführt werden.

In der Fig. 6 ist ein weiteres mögliches Ausführungsbeispiel der erfindungsgemäßen Magnetfeldtherapievorrichtung 1, welche den Magnetfeldgenerator 2 und die Matte 3 umfaßt, gezeigt. Dabei umfaßt eine Rechneranlage, insbesondere ein handelsüblicher PC 41 mit einem entsprechenden Softwareprogramm die Steuereinrichtung 4, welche einen digitalen Teil aufweist, welcher einen Mikrokontroller 7 umfaßt und mit einer Speichereinrichtung 8 zu einer dauerhaften Speicherung und einem bedarfsweisen Auslesen von Impulsmustern in Verbindung steht. Der PC 41 ist über die Anschlußleitungen 16 mit der Energiequelle 17 verbunden, wobei die Umwandlungseinrichtung 18 und der Transformator 19 vorzugsweise in einem Computernetzteil 42 integriert sind.

Die Speichereinrichtung 8 des Magnetfeldgenerators 2 wird in vorteilhafter Weise durch eine Festplatte 43 bzw. einen Arbeitsspeicher des PCs 41 gebildet.

Die Dateneingabe- bzw. Auswahleinrichtung 5 des Magnetfeldgenerators 2 kann in vorteilhafter Weise durch eine Tastatur 44 und/oder eine Maus 45 des PCs 41 gebildet sein und die Datenanzeige- bzw. Ausgabeeinrichtung 6 durch einen Bildschirm 46 bzw. Monitor realisiert sein.

Durch die Einbindung des PCs 41 wird es möglich, die Steuereinrichtung 4 durch hardwaremäßige Komponenten des PCs 41 bzw. ein entsprechendes Softwareprogramm auszubilden.

Weiters ist es ebenfalls möglich, die Empfangseinrichtung 12 und die Fernbedieneinrichtung 13, wie diese in den vorherigen Figuren beschrieben sind, zu verwenden. Die Fernbedieneinrichtung 13 kann wiederum durch eine Spracheingabeeinrichtung 14, welche ein Mikrofon 47 umfassen kann, oder mit dem externen Bedienelement 15 ausgeführt sein.

Die Signalübertragung zwischen dem externen Bedienelement 15 und der Empfangseinrichtung 12 kann dabei vorzugsweise über ein Infrarotsignal erfolgen. Zu diesem Zweck ist das externe Bedienelement 15 mit einem Infrarotsender und die Empfangseinrichtung 12 mit einem Infrarotempfänger ausgestattet.

Es ist aber ebenfalls möglich, die Signalübertragung zwischen der Fernbedieneinrichtung 13 und der Empfangseinrichtung 12 über Funk, Ultraschall oder dgl. durchzuführen.

Weiters ist es möglich, die Signalübertragung zwischen der Fernbedieneinrichtung 13 und der Empfangseinrichtung 12 auch mittels eines Kabels bzw. eines Bussystemes durchzuführen.

Weitere Komponenten des Magnetfeldgenerators 2, welche für gewöhnlich nicht in einem handelsüblichen PC integriert sind, wie beispielsweise die Empfangseinrichtung 12 für die Fernbedieneinrichtung 13 und/oder den Digital/Analogwandler 27 und/oder die Endstufe 28 und/oder die Regelung für den Heizungskreis 25 der Matte 3 und dgl., können vorzugsweise auf einer Schnittstellenkarte 48 aufgebaut sein.

Es ist jedoch ebenfalls möglich, handelsübliche Schnittstellenkarten 48, z.B. eine Audiokarte bzw. Soundkarte, welche die Funktion des Digital/Analogwandlers 27 und der Endstufe 28 übernimmt, zu verwenden und gegebenenfalls auf die Empfangseinrichtung 12 für die Fernbedieneinrichtung 13 und/oder die Regelung für den Heizungskreis 25 der Matte 3 zu verzichten.

Zum Aufbau des Magnetfeldgenerators 2 ist es somit lediglich notwendig, die Schnittstellenkarte 48 in einen freien Steckplatz, also eine freie Schnittstelle 49 im PC 41 einzubauen und das entsprechende Softwareprogramm, vorzugsweise auf der Festplatte 43, zu installieren.

Es ist jedoch ebenfalls möglich, den Digital/Analogwandler 27 und/oder die Endstufe 28 in einer externen Komponente 50 anzuordnen, die mit einer externen seriellen oder parallelen Schnittstelle 51 des PCs verbindbar ist, wie dies mit strichlierten Linien in Fig. 6 gezeigt ist.

Die Datenanzeige- bzw. Ausgabeeinrichtung 6 kann weiters, wie schon in den vorherigen Ausführungsvarianten beschrieben, eine Sprachausgabeeinrichtung 11 umfassen, welche zur Ausgabe von Sprachinformationen bzw. anderen akustischen Signalen geeignet ist. Diese Sprachausgabeeinrichtung 11 umfaßt vorzugsweise einen Lautsprecher 52 und kann auch zur Ausgabe von Musik verwendet werden.

Der Lautsprecher 52 kann mit dem PC oder der Schnittstellenkarte 48 oder der externen Komponente 50 des PCs 41 leitungsverbunden sein.

Zum kuppelbaren Anschluß zumindest einer Matte 3 kann die Schnittstellenkarte 48 oder die externe Komponente 50 zumindest eine Ausgangsbuchse 20 aufweisen. Die Leitung 22 bzw. die Leitungen 22 der Matte 3 sind mit einem korrespondierenden Stecker 21 ausgestattet. Wird als Schnittstellenkarte 48, welche die Endstufe 28 umfaßt, eine handelsübliche Soundkarte verwendet, so ist es vorteilhaft, den korrespondierenden Stecker 21 als 3,5 mm Klinkenstecker auszubilden.

Es ist aber ebenso möglich, zwischen der bzw. den Ausgangsbuchsen 20 und den Steckern 21 einen zusätzlichen Adapter 53 anzuordnen, welcher die Möglichkeit bietet, mehrere Matten 3 mit mehreren Steckern 21 an einer Ausgangsbuchse 20 anzuschließen. Die Verdrahtung in diesem zusätzlichen Adapter kann so ausgebildet sein, daß sich daraus sowohl eine serielle Schaltung der Matten 3 als auch eine Parallelschaltung der Matten 3 ergibt. Durch die Verwendung des zusätzlichen Adapters kann die Möglichkeit geschaffen werden, mehrere Matten 3 mit einem Magnetfeldgenerator 2 zu betreiben, welche gleichzeitig mit Energie beaufschlagbar sind.

Selbstverständlich ist es aber auch möglich, an der Schnittstellenkarte 48 oder der externen Komponente 50 mehrere Ausgangsbuchsen 20 vorzusehen, um damit die Möglichkeit zu schaffen, mehrere Matten 3 zu versorgen.

Um eine Leistungsausbeute des Magnetfeldgenerators 2 zu erhöhen, ist es möglich, die Energiebeaufschlagung der einzelnen Matten 3 zeitlich voneinander versetzt durchzuführen, d.h. also, während die eine Matte 3 mit Energie beaufschlagt wird, befindet sich die andere Matte 3 gerade in einem Zeitabschnitt, in dem keine Energie zugeführt wird. Auf diese Weise ist es beispielsweise möglich, Leistungsbauteile des Magnetfeldgenerators 2 für zwei Matten 3 auszulegen und drei Matten 3 zu betreiben.

Die für die Magnetfeldtherapie wichtigen Wellenformen, wie diese schon in den Fig. 3 bis Fig. 5 näher beschrieben sind, können von einer Diskette, einer CD, einer DVD durch ein Laufwerk 54 geladen und in die Speichervorrichtung 8, insbesondere die Festplatte 43, eingelesen werden. Es ist aber ebenfalls möglich, neue Wellenformen bzw. Softwareupdates über ein Internet 55 zu übertragen.

Der Ordnung halber sei abschließend darauf hingewiesen, daß zum besseren Verständnis der Magnetfeldtherapievorrichtung 1 diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Vor allem können die einzelnen in den Fig. 1; 2; 3; 4; 5; 6 gezeigten Ausführungen und Maßnahmen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Magnetfeldtherapievorrichtung
- 2: Magnetfeldgenerator
- 3: Matte
- 4: Steuereinrichtung
- 5: Dateneingabe- bzw. Auswahleinrichtung

- 6: Datenanzeige- bzw. Ausgabeeinrichtung
- 7: Mikrokontroller
- 8: Speichereinrichtung
- 9: Tastenfeld
- 10: LCD-Display

- 11: Sprachausgabeeinrichtung
- 12: Empfangseinrichtung
- 13: Fernbedieneinrichtung
- 14: Spracheingabeeinrichtung
- 15: externes Bedienelement

- 16: Anschlußleitung
- 17: Energiequelle
- 18: Umwandlungseinrichtung
- 19: Transformator
- 20: Ausgangsbuchse

- 21: Stecker
- 22: Leitung
- 23: Spule
- 24: Dauermagnet
- 25: Heizungskreis

- 26: Temperatursensor
- 27: Digital/Analogwandler
- 28: Endstufe
- 29: Impuls
- 30: Pause

- 31: erste Impulsgruppe
- 32: Pause
- 33: zweite Impulsgruppe
- 34: Pause
- 35: dritte Impulsgruppe

- 36: Pause
- 37: Schwingung
- 38: Amplitudenhöhe
- 39: Amplitudenhöhe
- 40: Schnittstelle

- 41: PC
- 42: Computernetzteil
- 43: Festplatte
- 44: Tastatur
- 45: Maus

- 46: Bildschirm
- 47: Mikrofon
- 48: Schnittstellenkarte
- 49: Schnittstelle
- 50: Komponente

- 51: Schnittstelle
- 52: Lautsprecher
- 53: Adapter
- 54: Laufwerk
- 55: Internet

## Patentansprüche

1. Magnetfeldgenerator für zumindest eine in oder auf einer Matte angeordneten Spule zur Behandlung von menschlichem und/oder tierischem und/oder pflanzlichem Gewebe, welcher eine Steuereinrichtung zur Erzeugung eines pulsierenden Stromes umfaßt und an einer Energiequelle angeschlossen ist, **dadurch gekennzeichnet, daß** die Steuereinrichtung (4) einen digitalen Teil aufweist, welcher einen Mikrokontroller (7) umfaßt, welcher seinerseits eine Speichereinrichtung (8) zu einer dauerhaften Speicherung und einem bedarfsweisen Auslesen von Impulsmustern umfaßt bzw. mit dieser in Verbindung steht und die Steuereinrichtung (4) mit einer Dateneingabe- bzw. Auswahleinrichtung (5), einer Datenanzeige- bzw. Ausgabeeinrichtung (6) und einer Empfangseinrichtung (12) für eine Fernbedieneinrichtung (13) in Verbindung steht und ausgangsseitig einen Digital/Analogwandler (27) aufweist, welcher mit einer Endstufe (28) verbunden ist, welche ihrerseits ausgangsseitig mit zumindest einer Matte (3) verbindbar ist.

2. Magnetfeldgenerator für zumindest eine in oder auf einer Matte angeordneten Spule zur Behandlung von menschlichem und/oder tierischem und/oder pflanzlichem Gewebe, welcher eine Steuereinrichtung zur Erzeugung eines pulsierenden Stromes umfaßt und an einer Energiequelle angeschlossen ist, **dadurch gekennzeichnet, daß** eine Rechneranlage, insbesondere ein handelsüblicher PC (41), mit einem entsprechenden Softwareprogramm die Steuereinrichtung (4), welche einen digitalen Teil aufweist, welcher einen Mikrokontroller (7) umfaßt und eine Speichereinrichtung (8) zu einer dauerhaften Speicherung und einem bedarfsweisen Auslesen von Impulsmustern umfaßt und die Steuereinrichtung (4) bzw. der PC (41) mit einer Dateneingabe- bzw. Auswahleinrichtung (5) und einer Datenanzeige- bzw. Ausgabeeinrichtung (6) in Verbindung steht und über eine Schnittstelle (49, 51) mit einen Digital/Analogwandler (27), welcher mit einer Endstufe (28) verbunden ist, welche ihrerseits ausgangsseitig mit zumindest einer Matte (3) verbindbar ist, leitungsverbunden ist.

3. Magnetfeldgenerator nach Anspruch 2, **dadurch gekennzeichnet, daß** die Speichereinrichtung (8) durch eine Festplatte (43) des PCs (41) gebildet ist.

4. Magnetfeldgenerator nach einem oder mehreren der Ansprüche 2 oder 3, **dadurch gekennzeichnet, daß** die Dateneingabe- bzw. Auswahleinrichtung (5) durch eine Tastatur (44) und/ oder eine Maus (45) des PCs (41) gebildet ist.

5. Magnetfeldgenerator nach einem oder mehreren der Ansprüche 2 bis 4, **dadurch gekennzeichnet, daß** die Datenanzeige- bzw. Ausgabeeinrichtung (6) durch einen Bildschirm (46) bzw. einen Monitor des PCs (41) gebildet ist.

6. Magnetfeldgenerator nach einem oder mehreren der Ansprüche 2 bis 5, **dadurch gekennzeichnet, daß** die Schnittstelle (49) im PC (41) angeordnet ist und zur Aufnahme einer Schnittstellenkarte (48) dient, welche den Digital/Analogwandler (27) und/oder die Endstufe (28) umfaßt.

7. Magnetfeldgenerator nach Anspruch 6, **dadurch gekennzeichnet, daß** die Schnittstellenkarte (48) als handelsübliche Audiokarte bzw. Soundkarte ausgebildet ist.

8. Magnetfeldgenerator nach einem oder mehreren der Ansprüche 2 bis 5, **dadurch gekennzeichnet, daß** die Schnittstelle (51) als externe parallele oder serielle Schnittstelle (47) des PCs (41) ausgebildet ist und zur Verbindung mit einer externen Komponente (50), welche den Digital/Analogwandler (27) und/oder die Endstufe (28) umfaßt, ausgebildet ist.

9. Magnetfeldgenerator nach einem oder mehreren der Ansprüche 2 bis 8, **dadurch gekennzeichnet, daß** die Speichereinrichtung (8) bzw. das Softwareprogramm zu einen Einlesen und/oder einem Speichern weiterer Impulsmuster, welche von einem Laufwerk (54), insbesondere einem Diskettenlaufwerk und/oder einem CD-ROM-Laufwerk und/ oder einem DVD-Laufwerk und/oder einem Internet (55) übermittelbar sind, ausgebildet ist.

10. Magnetfeldgenerator nach einem oder mehreren der Ansprüche 2 bis 6 und 9, **dadurch gekennzeichnet, daß** die Schnittstellenkarte (48) mit einer Empfangseinrichtung (12) für eine Fernbedieneinrichtung (13) ausgestattet ist.

11. Magnetfeldgenerator nach einem oder mehreren der Ansprüche 2 bis 5 und 8 bis 9, **dadurch gekennzeichnet, daß** die externe Komponente (50), welche den Digital/ Analogwandler (27) und/oder die Endstufe (28) umfaßt, mit einer Empfangseinrichtung (12) für eine Fernbedieneinrichtung (13) ausgestattet ist.

12. Magnetfeldgenerator nach einem oder mehreren der vorhergehenden Ansprüche, **dadurch gekennzeichnet, daß** die Speichereinrichtung (8) zur dauerhaften Speicherung von werksseitig fix hinterlegten und mittels der Dateneingabe- bzw. Auswahleinrichtung (5) frei programmierbaren Impulsmustern ausgebildet ist.

13. Magnetfeldgenerator nach Anspruch 1 oder 12, **dadurch gekennzeichnet, daß** die Steuereinrichtung (4) mit einer Schnittstelle (40) zu einem Ändern und/oder Ergänzen der werksseitig fix in der Speichereinrichtung (8) hinterlegten Impulsmuster mittels eines Softwareupdates verbunden ist.

14. Magnetfeldgenerator nach einem oder mehreren der vorhergehenden Ansprüche, **dadurch gekennzeichnet, daß** die werksseitig fix in der Speichereinrichtung (8) hinterlegten Impulsmuster vorzugsweise in einer vierfachen Verschachteln von ein oder mehreren Impulsen (29) und dazwischen angeordneten Pausen (30), welche zu einer ersten Impulsgruppe (31) zusammengefaßt sind und ein oder mehreren dieser ersten Impulsgruppen (31) und dazwischen angeordneten Pausen (32), welche zu einer zweiten Impulsgruppe (33) zusammengefaßt sind und ein oder mehreren dieser zweiten Impulsgruppen (33) und dazwischen angeordneten Pausen (34), welche zu einer dritten Impulsgruppe (35) zusammengefaßt sind und ein oder mehreren dieser dritten Impulsgruppen (35) und dazwischen angeordneten Pausen (36) ausgebildet sind.

15. Magnetfeldgenerator nach einem oder mehreren der vorhergehenden Ansprüche, **dadurch gekennzeichnet, daß** die mittels der Dateneingabe- bzw. Auswahleinrichtung (5) frei programmierbaren Impulsmuster vorzugsweise in einer vierfachen Verschachteln von ein oder mehreren Impulsen (29) und dazwischen angeordneten Pausen (30), welche zur ersten Impulsgruppe (31) zusammengefaßt sind und ein oder mehreren dieser ersten Impulsgruppen (31) und dazwischen angeordneten Pausen (32), welche zur zweiten Impulsgruppe (33) zusammengefaßt sind und ein oder mehreren dieser zweiten Impulsgruppen (33) und dazwischen angeordnete Pausen (34), welche zur dritten Impulsgruppe (35) zusammengefaßt sind und mehreren dieser dritten Impulsgruppen (35) und dazwischen angeordneten Pausen (36) ausgebildet sind.

16. Magnetfeldgenerator nach Anspruch 14, **dadurch gekennzeichnet, daß** die Verschachteln der fix in der Speichereinrichtung (8) hinterlegten und von dieser in den Mikrokontroller (7) bzw. die Steuereinrichtung (4) einlesbaren Impulsmuster ein- bis achtfach, vorzugsweise vierfach ist.

17. Magnetfeldgenerator nach Anspruch 15, **dadurch gekennzeichnet, daß** die Verschachtelung der frei mittels der Dateneingabe- bzw. Auswahleinrichtung (5) frei programmierbaren Impulsmuster ein- bis achtfach, vorzugsweise vierfach ist.

18. Magnetfeldgenerator nach einem oder mehreren der vorhergehenden Ansprüche, **dadurch gekennzeichnet, daß** die Anzahl der Impulse (29) und der dazwischen angeordneten Pausen (30), welche zur ersten Impulsgruppe (31) zusammengefaßt sind, zwischen 1 und 100 ist.

19. Magnetfeldgenerator nach einem oder mehreren der vorhergehenden Ansprüche, **dadurch gekennzeichnet, daß** die Anzahl der ersten Impulsgruppen (31) und der dazwischen angeordneten Pausen (32), welche zur zweiten Impulsgruppe (33) zusammengefaßt sind, zwischen 1 und 100 ist.

20. Magnetfeldgenerator nach einem oder mehreren der vorhergehenden Ansprüche, **dadurch gekennzeichnet, daß** die Anzahl der zweiten Impulsgruppen (33) und der dazwischen angeordneten Pausen (34), welche zur dritten Impulsgruppe (35) zusammengefaßt sind, zwischen 1 und 100 ist.

21. Magnetfeldgenerator nach einem oder mehreren der vorhergehenden Ansprüche, **dadurch gekennzeichnet, daß** die Anzahl der dritten Impulsgruppen (35) und der dazwischen angeordneten Pausen (36) zwischen 1 und 100 ist.

22. Magnetfeldgenerator nach einem oder mehreren der vorhergehenden Ansprüche, **dadurch gekennzeichnet, daß** bei einer höheren Verschachtelung der Impulse (29) bzw. der ersten, zweiten und dritten Impulsgruppen (31, 33, 35) die Anzahl einer höheren Impulsgruppe, welche mit darauffolgenden Pausen zu einer noch höheren Impulsgruppe zusammengefaßt ist, jeweils zwischen 1 und 100 ist.

23. Magnetfeldgenerator nach einem oder mehreren der vorhergehenden Ansprüche, **dadurch gekennzeichnet, daß** nach ein oder mehreren der dritten Impulsgruppen (35) und der dazwischen angeordneten Pausen (36) die Polarität der Impulse (29) umkehrbar ist.

24. Magnetfeldgenerator nach einem oder mehreren der vorhergehenden Ansprüche, **dadurch gekennzeichnet, daß** je nach der Höhe der Verschachtelung nach ein oder mehreren der höchsten Impulsgruppen und der dazwischen angeordneten Pausen die Polarität der Impulse (29) umkehrbar ist.

25. Magnetfeldgenerator nach einem oder mehreren der vorhergehenden Ansprüche, **dadurch gekennzeichnet, daß** die Impulse (29) dreieckförmig bzw. sägezahnförmig ausgebildet sind.

26. Magnetfeldgenerator nach einem oder mehreren der vorhergehenden Ansprüche, **dadurch gekennzeichnet, daß** zumindest der jeweils erste und letzte Impuls (29) der ersten Impulsgruppe (31) mit einer niedrigeren Amplitudenhöhe (38) ausgebildet sind und alle dazwischenliegenden Impulse (29) der ersten Impulsgruppe (30) mit einer höheren Amplitudenhöhe (39) ausgebildet sind.

27. Magnetfeldgenerator nach einem oder mehreren der vorhergehenden Ansprüche, **dadurch gekennzeichnet, daß** eine steigende Flanke der Impulse (29) mit einer aus positiven Halbwellen bestehenden Schwingung (37) überlagert ausgebildet ist (Betriebszustand "Yang").

28. Magnetfeldgenerator nach einem oder mehreren der vorhergehenden Ansprüche, **dadurch gekennzeichnet, daß** eine steigende Flanke der Impulse (29) mit einer aus negativen Halbwellen bestehenden Schwingung (37) überlagert ausgebildet ist (Betriebszustand "Yin").

29. Magnetfeldgenerator nach einem oder mehreren der vorhergehenden Ansprüche, **dadurch gekennzeichnet, daß** die Anzahl der positiven bzw. negativen Schwingungen (37), mit welcher die steigende Flanke des Impulses (29) überlagert ist, zwischen 1 und 100 ist.

30. Magnetfeldgenerator nach einem oder mehreren der vorhergehenden Ansprüche, **dadurch gekennzeichnet, daß** die Impulse (29) mit einer Frequenz von 0,02 Hz bis 80 kHz ausgebildet sind.

31. Magnetfeldgenerator nach einem oder mehreren der vorhergehenden Ansprüche, **dadurch gekennzeichnet, daß** ein Verhältnis zwischen dem Impuls (29) und der Pause (30) und zwischen der ersten Impulsgruppe (31) und der Pause (32) und zwischen der zweiten Impulsgruppe (33) und der Pause (34) und zwischen der dritten Impulsgruppe (35) und der Pause (36) 1,618 ist (goldener Schnitt).

32. Magnetfeldgenerator nach einem oder mehreren der vorhergehenden Ansprüche, **dadurch gekennzeichnet, daß** die Speichereinrichtung (8) zur Speicherung von Sprachinformationen in digitaler Form für die Sprachausgabeeinrichtung (11) ausgebildet ist.

33. Magnetfeldgenerator nach einem oder mehreren der vorhergehenden Ansprüche, **dadurch gekennzeichnet, daß** die Speichereinrichtung (8) zur Speicherung von Sprachinformationen verschiedener Sprachen in digitaler Form für eine Ausgabe von verschiedenen Sprachen durch die Sprachausgabeeinrichtung (11) ausgebildet ist.

34. Magnetfeldgenerator nach einem oder mehreren der Ansprüche 1 und 12 bis 33, **dadurch gekennzeichnet, daß** die Dateneingabe- bzw. Auswahleinrichtung (5) durch Taster bzw. ein Tastenfeld (9) aufgebaut ist.

35. Magnetfeldgenerator nach einem oder mehreren der Ansprüche 1 und 12 bis 34, **dadurch gekennzeichnet, daß** die Datenanzeige- bzw. Ausgabeeinrichtung (6) durch ein LCD-Display (10) aufgebaut ist.

36. Magnetfeldgenerator nach einem oder mehreren der vorhergehenden Ansprüche, **dadurch gekennzeichnet, daß** die Dateneingabe- bzw. Auswahleinrichtung (5) und die Datenanzeige- bzw. Ausgabeeinrichtung (6) als Touch-Screen ausgebildet sind.

37. Magnetfeldgenerator nach einem oder mehreren der vorhergehenden Ansprüche, **dadurch gekennzeichnet, daß** die Fernbedieneinrichtung (13) durch eine Spracheingabeeinrichtung (14) ausgebildet ist und die Empfangseinrichtung (13) ein Mikrofon umfaßt.

38. Magnetfeldgenerator nach einem oder mehreren der vorhergehenden Ansprüche, **dadurch gekennzeichnet, daß** die Fernbedieneinrichtung (13) durch ein externes Bedienelement (15) ausgebildet ist.

39. Magnetfeldgenerator nach Anspruch 38, **dadurch gekennzeichnet, daß** das externe Bedienelement (15) ein Display umfaßt.

40. Magnetfeldgenerator nach Anspruch 38, **dadurch gekennzeichnet, daß** das externe Bedienelement (15) mit einem Infrarotsender und die Empfangseinrichtung (12) mit einem Infrarotempfänger ausgebildet ist.

41. Magnetfeldgenerator nach Anspruch 38, **dadurch gekennzeichnet, daß** eine Signalübertragung zwischen dem externen Bedienelement (15) und der Empfangseinrichtung (12) mittels Funk ausgebildet ist.

42. Magnetfeldgenerator nach Anspruch 38, **dadurch gekennzeichnet, daß** eine Signalübertragung zwischen dem externen Bedienelement (15) und der Empfangseinrichtung (12) mittels einer Leitung ausgebildet ist.

43. Magnetfeldgenerator nach einem oder mehreren der vorhergehenden Ansprüche, **dadurch gekennzeichnet, daß** die Endstufe (28) ausgangsseitig zumindest eine Ausgangsbuchse (17) zum kuppelbaren Anschluß zumindest einer Leitung (22) für zumindest eine Matte (3) aufweist.

44. Magnetfeldgenerator nach einem oder mehreren der vorhergehenden Ansprüche, **dadurch gekennzeichnet, daß** am Ausgang der Endstufe (28) mehrere Ausgangsbuchsen (20), welche gleichzeitig oder zeitlich versetzt mit Energie beaufschlagbar sind, angeordnet sind.

45. Magnetfeldgenerator nach einem oder mehreren der vorhergehenden Ansprüche, **dadurch gekennzeichnet, daß** die Steuereinrichtung (4) zur Steuerung und/oder Regelung zumindest einer Temperatur zumindest eines Heizungskreises (25) von zumindest einer Matte (3), welche über zumindest eine Leitung (22) und zumindest eine Ausgangsbuchse (20) mit der Steuereinrichtung (4) verbunden ist/sind, ausgebildet ist.

46. Matte zur Behandlung von menschlichem und/oder tierischem und/oder pflanzlichem Gewebe mit Hilfe eines pulsierenden Magnetfeldes, **dadurch gekennzeichnet, daß** eine Matte (3) zumindest eine Spule (23) und zumindest einen Heizungskreis (25) aufweist.

47. Matte nach Anspruch 46, **dadurch gekennzeichnet, daß** die Spule (23) mit einer Windungszahl von 10 bis 40 Windungen ausgebildet ist.

48. Matte nach den Ansprüchen 46 oder 47, **dadurch gekennzeichnet, daß** die Spule (23) für einen Strom bis etwa 1,5 Ampere ausgebildet ist.

49. Matte nach einem oder mehreren der Ansprüche 46 bis 48, **dadurch gekennzeichnet, daß** die Matte (3) für den Einsatz in einem Kraftfahrzeug als Sitzüberzug ausgebildet ist.

50. Matte nach einem oder mehreren der Ansprüche 46 bis 49, **dadurch gekennzeichnet, daß** zur Bestimmung bzw. zur Regelung der Temperatur des Heizungskreises (25) in der Matte (3) ein Temperatursensor (26) eingearbeitet ist.

51. Matte nach ein oder mehreren der Ansprüche 46 bis 50, **dadurch gekennzeichnet, daß** im Zentrum der Spule (23) bzw. der Spulen (23) jeweils ein Dauermagnet (24) angeordnet ist.

52. Matte nach einem oder mehreren der Ansprüche 46 bis 51, **dadurch gekennzeichnet, daß** die Matte (3) zur Aufnahme eines Magnetfeldgenerators (2) ausgebildet ist.

53. Magnetfeldtherapievorrichtung zur Behandlung von menschlichem und/oder tierischem und/oder pflanzlichem Gewebe, welche einen Magnetfeldgenerator und eine Matte umfaßt, **dadurch gekennzeichnet, daß** der Magnetfeldgenerator nach den Ansprüchen 1 bis 45 und die Matte nach den Ansprüchen 46 bis 52 ausgebildet ist.
